# EUROPEAN PATENT SPECIFICATION

(11) **EP 4 096 536 B1**
(45) Date of publication and mention of the grant of the patent: **28.02.2024**
(21) Application number: 21706709.9
(22) Date of filing: 28.01.2021
(51) Int. Cl.: A61B 17/16

(54) **TOOL FOR THE SELECTIVE SECTION OF TRANSVERSE PROCESSES OF THE CERVICAL VERTEBRAE**
WERKZEUG ZUR SELEKTIVEN SEZIERUNG VON QUERFORTSÄTZEN DER HALSWIRBEL
OUTIL POUR LA SECTION SÉLECTIVE D'APOPHYSES TRANSVERSES DES VERTÈBRES CERVICALES

(30) Priority: 29.01.2020 IT 202000001702
(43) Date of publication of application: 07.12.2022
(73) Proprietor: Università degli Studi di Padova, 35122 Padova (IT)
(72) Inventor: DE CARO, Raffaele, 35122 Padova (IT); BOSCOLO BERTO, Rafael, 35122 Padova (IT); MACCHI, Veronica, 35122 Padova (IT); PORZIONATO, Andrea, 35122 Padova (IT)
(74) Representative: Di Bernardo, Antonio
(86) International application number: PCT/IB2021/050649
(87) International publication number: WO 2021/152486

(56) References cited:
- US-A1- 2007 016 210
- US-A1- 2011 040 301
- US-A1- 2012 010 622

## Description

### TECHNICAL FIELD

The present invention relates to the field of tools for medical/veterinary applications. In particular, the invention relates to a tool for performing the selective section of transverse processes of the cervical vertebrae, that is, an invasive procedure to allow the exposure and access to the artery and to the vertebral veins running within the transverse foramen.

### BACKGROUND

In the areas of clinical-surgical didactics relative to the skull-cervical district, of legal medicine, forensic clinical anatomy, scientific experimentation and research with respect to both the medical science and the veterinary science, there is a need to access the cervical anatomical district with particular reference to the vertebral vessels.

Said procedure expresses a polyhedral value, of clinical-surgical, medical-legal and research didactics, moreover both in the human and animal field.

From a didactic point of view, the medical student and the training specialist benefit from studies on the corpse for learning anatomy (see [1], [2] and [3]). In particular, surgical educational programmes consisting of cadaveric models have gained popularity in recent years, enabling common and innovative surgical techniques to be performed (see [4]), which, by simulating a real surgical setting, allows complex surgical skills to be acquired (see [5]).

From a medical and legal point of view, the importance of forensic clinical anatomy has been recognized for some time (see [6]) and integrated into the guidelines of the medicolegal investigation in relation to the alleged medical professional responsibility. In particular, autopsy examination of the cervical tract is highly recommended for the assessment of neurological and vascular diseases (see [7] and [8]), in order to identify and diagnose diseases that directly/indirectly affect the central nervous system. It enables to provide detailed descriptions on the lesions found in relation to the thus isolated structural and functional systems (see [9]).

Finally, even basic researchers and neuroscientists increasingly require access to specific cervical districts with increasing experimental scientific interest.

In particular, the vertebral vessels (vertebral artery and vertebral vein) pass through the transverse foramina present in the transverse processes of the cervical vertebrae. Consequently, an invasive procedure is necessary to allow access to a transverse foramen and expose the vertebral vessels surrounded by the transverse processes.

Nonetheless, there are no tools dedicated to this type of section procedure, in the art it is usual to resort to a series of devices designed for different purposes.

For example, in the art electric oscillating saws with circumferential or fan blade, surgical scissors of variable strength, bone tweezers and pincers (see [10], [11]), or also surgical chisels known by the name of rachiotomes actually aimed at the isolation of the spinal cord contained in the lower spinal sections (e.g. thoracic and lumbar) with a coarse, invasive and poorly directional/controllable tool use approach (see [12], [13] and [14]) are used to perform this section procedure.

Moreover, although various surgical tools, called osteotomes designed to cut or incise a bone (see [15], [16], [17], [18], [19], [20]) have been proposed in the art, none of these devices is suitable for the above section procedure.

Consequently, the effectiveness of such tools for this purpose is substantially limited and the precision of execution of such procedures is substantially compromised, even if it is made with devices directed to the generally vertebral sphere.

In fact, during the section procedure there is a very high probability of causing lesion with the tool of the structures of interest contained within the transverse foramina of the cervical vertebrae, even when carried out by an expert operator.

The damage to such structures of interest reduces or even cancels out the clinical-surgical, medical-legal/forensic or research didactic usefulness in both human and veterinary fields. In particular, the possibilities of interpreting the physiopathological nature of what has been observed - for example, anatomical variant, pathology, outcome of trauma, isolation error in dissection - during the course of the carried out activities are variously compromised up to the extreme degrees.

Therefore, in the art there is a need for a tool which allows easy access to the cervical anatomical district, with particular reference to the transverse foramen and to the vertebral vessels contained therein, allowing to preserve such structures of interest in their morphological integrity during surgical-septal isolation.

### REFERENCES

*[1]* Macchi V, Munari PF, Ninfo V, Parenti A, De Caro R. 2003. A short course of dissection for second-year medical students at the School of Medicine of Padova. Surg Radiol Anat 25:132-138*.*
[2] Macchi V, Porzionato A, Stecco C, Parenti A, De Caro R. 2007. Clinical neuroanatomy module 5 years' experience at the School of Medicine of Padova. Surg Radiol Anat 29:261-267.
[3] De Caro R, Macchi V, Porzionato A. 2009. Promotion of body donation and use of cadavers in anatomical education at the University of Padova. Anat Sci Educ 2:91-92*.*
[4] Harrop J, Rezai AR, Hoh DJ, Ghobrial GM, Sharan A. 2013. Neurosurgical training with a novel cervical spine simulator: Posterior foraminotomy and laminectomy. Neurosurgery 73:94-99*.*
[5] Tortolani PJ, Moatz BW, Parks BG, Cunningham BW, Sefter J, Kretzer RM. 2014. Cadaver training module for teaching thoracic pedicle screw placement to residents. Orthopedics 36:1128-1133*.*
[6] Porzionato A, Macchi V, Stecco C, Loukas M, Tubbs RS, De Caro R. 2017. Forensic clinical anatomy: A new field of study with application to medicolegal issues. Clin Anat 30:2-5*.*
[7] Brinkmann B. 1999. Harmonisation of medico-legal autopsy rules. Int J Legal Med 113:1-14.
[8] Ferrara SD, Baccino E, Bajanowski T, Boscolo-Berto R, Castellano M, Angel R De, Pauliukevičius A, Ricci P, Vanezis P, Vieira DN, Viel G, Villanueva E. 2013. Malpractice and medical liability. European Guidelines on methods of ascertainment and criteria*.*
[9] Powers JM. 1995. Practice guidelines for autopsy pathology. Autopsy procedures for brain, spinal cord, and neuromuscular system. Autopsy Committee of the College of American Pathologists. Arch Pathol Lab Med 119:777-783*..*
[10] Sohn D. 1967. Removal of complete spinal cord at autopsy. Am J Clin Pathol 47:649-651*.*
[11] Walker JEC, Rutty GN, Rodgers B, Woodford NWF. 2002. How should the chest wall be opened at necropsy? J Clin Pathol 55:72-75*..*
[12] Brunetti L. 1863. Sopra il nuovo rachiotomo e sul metodo d'aprire lo speco vertebrate. Padova. 84-111 p*.*
[13] Hektoen L. 1894. The technic of medicolegal post-mortem examination. Chicago, USA: Keener Company. 172 p*.*
*[14]* Kiæer W, Olsen. A. 1950. A new technique for the removal of the spinal cord at autopsy. Acta Pathol Microbiol Scand 497-500*.*
[15] WO 2018/040918*.*
[16] CN 205054368*.*
[17] CN 204971449.
[18] CN 204072218*.*
[19] CN 203369939*.*
[20] US 5,722,977*.*

Document US2007/016210A1 discloses a tool according to the preamble of claim 1.

### OBJECTS AND SUMMARY OF THE INVENTION

An object of the present invention is to overcome the disadvantages of the prior art.

In particular, it is an object of the present invention to provide a tool for the selective section of the transverse processes of the cervical vertebrae or 'transversoclasiotome', which allows to preserve intact the structures of interest contained within the transverse foramina of the cervical vertebrae, in particular the cervical vessels.

These and other objects of the present invention are achieved by a device incorporating the features of the accompanying claims, which form an integral part of the present description.

According to one aspect of the present invention, the tool comprises a first arm and a second arm. In turn, each arm comprises a handle and a beak aligned along a main extension direction of the tool. The first arm and the second arm are rotatably coupled one to the other by means of a pin in order to rotate in a rotation plane between a first configuration in which the beak of the first arm is in contact with the beak of the second arm and a second configuration in which the beak of the first arm is spaced from the beak of the second arm.

In particular, the beak of the first arm comprises a cutting portion, said cutting portion having a wedge-shaped section in a section plane transversal to the rotation plane of the arms, and the beak of the second arm comprises an abutment portion having a receptacle configured to receive a cutting end of the cutting portion when the beak of the first arm is in contact with the beak of the second arm in the second configuration. Advantageously, the receptacle of the abutment portion comprises a through slot configured to receive the cutting end of the cutting portion for its entire dimension of length.

Thanks to this solution, it is possible to perform a section of the transverse processes in a simple and effective way. In particular, the receptacle of the abutment portion receiving the cutting end of the cutting portion allows to obtain a precise sectioning and an easily control of the transverse process and at the same time it allows the cutting portion to act as a separator wedge during the sectioning of the transverse process, separating the bone margins generated by the section in a reliable manner.

In particular, the presence of the slot prevents, once the incision has been completed, the cutting end from interacting in an uncontrolled manner with the surrounding bone, nerve and/or tissue structures.

In one embodiment, both the cutting portion and the receptacle of the abutment portion have a symmetrical structure with respect to the rotation plane of the arms of the tool.

Thanks to this solution it is possible to obtain a symmetrical distribution of the forces on the arms of the tool, in particular on the cutting portion and on the abutment portion, during the sectioning of the resistant bone structure. As a result, it is possible to have greater control of the tool during sectioning. Moreover, this solution allows ergonomic use both with right-handed and left-handed operators.

In one embodiment, the cutting portion comprises a pair of side surfaces which joins together to define the cutting end. When the beak of the first arm is in contact with the beak of the second arm in the second position, said lateral surfaces are configured to come into contact with an internal side wall of the abutment portion, said internal side wall delimiting the receptacle.

Even more preferably, a profile of each of said side surfaces of the cutting portion has a convexity in a position proximal to the cutting end. In this case, the contact between the side surfaces of the cutting portion and the side wall of the abutment portion occurs in correspondence of said convexity.

Through this structure, the cutting end of the cutting portion is free to completely cross the resistant bone structure of the transverse process when the abutment portion and the cutting portion are fully clamped together. Consequently, a particularly sharp and precise sectioning of the transverse process is obtained. In this way, however, the wear of the cutting edge is limited, since the latter is prevented from coming into contact against a plane.

In one embodiment, the receptacle comprises a through slot comprising a first opening exposed on the abutment portion facing the beak of the first arm and a second opening exposed on the abutment portion opposite to the first opening. When the beak of the first arm is in contact with the beak of the second arm in the second configuration, the cutting end of the cutting portion does not protrude beyond the second opening of the through slot.

Thanks to this solution, the second opening allows the outflow of any fluids and/or bone or tissue micro-fragments from the region below the cutting end of the cutting portion during the sectioning of the transverse process, increasing the precision and reliability of the tool.

In one embodiment, the cutting portion and the abutment portion have a dimension of length that extends along a secondary extension direction inclined at an angle included between 15° and 45°, preferably 30°, with respect to the main extension direction of the tool.

In this way, a high degree of ergonomics is obtained in the application context, taking into account that the corpse/patient is typically in supine position, while the operator is in upright position at his side.

In one embodiment, the beak of the first arm comprises a connecting portion connected to the cutting portion and proximal to the handle of the first arm. Similarly, the beak of the second arm comprises a connecting portion connected to the abutment portion and proximal to the handle of the second arm. Said connecting portions have a dimension of length that extends along the main extension direction.

The clamping system defined by the beaks thus formed brings the cutting portion and the abutment portion to close, approaching one another almost parallel - in a 'guillotine' manner - in place of a more common 'shear' or 'scissors' type closure. This allows to limit the sliding of the tool during the sectioning of the resistant bone structure of the transverse process, preventing the latter from escaping from the front of the tool.

In one embodiment, the first arm comprises a pair of coupling portions interposed between the beak and the handle. Similarly, the second arm comprises a single coupling portion interposed between the beak and the handle, wherein the two coupling portions of the first arm are spaced apart so as to define a housing configured to receive the coupling portion of the second arm, and wherein each coupling portion comprises a through hole configured to be coaxial with the through holes of the other coupling portions and for receiving said pin in a rotatable manner.

Thanks to this structure, the possibility that the cutting end of the cutting portion is deviated from the rotation plane during the clamping of the beaks due to the resistance of the bone structure of the transverse process is reduced, or completely prevented. Consequently, the sectioning operation is easier to perform for the operator.

In one embodiment, the abutment portion comprises a beveled or rounded free end.

Thanks to this solution, it is avoided that during the introduction of the abutment portion into the transverse foramen the structures contained therein and/or the adjacent structures are damaged.

In one embodiment, both the cutting portion and the abutment portion have a maximum width dimension, lying in the section plane, between 1.5 mm and 5 mm, preferably equal to 2 mm.

Preferably, and the abutment portion is made with a maximum length dimension, lying in the rotation plane, substantially comprised between 12 mm and 15 mm, more preferably substantially equal to 13 mm. In addition or alternatively, the cutting portion has a maximum length dimension, lying in the rotation plane ρ, substantially comprised between 8 mm and 11 mm, more preferably substantially equal to 10 mm.

Moreover, the abutment portion preferably has a maximum depth dimension parallel to the rotation plane between 1.5 mm and 1 mm, more preferably equal to 1.2 mm. In addition or alternatively, the cutting portion has a dimension of maximum depth parallel to the rotation plane comprised between 5 mm and 6.5 mm, preferably equal to 6 mm.

In one embodiment, the cutting portion comprises a removable blade and a receptacle integral with the beak, wherein the removable blade is coupled in a removable manner with the receptacle.

In this way it is possible to replace only the blade when it is worn and/or damaged, thus guaranteeing a prolonged life of the tool as a whole.

Further features and advantages of the present invention will be more apparent from the description of the accompanying drawings.

### BRIEF DESCRIPTION OF THE DRAWINGS

The invention will be described below with reference to some examples, provided for explanatory and non-limiting purposes, and illustrated in the accompanying drawings. These drawings illustrate different aspects and embodiments of the present invention and, where appropriate, reference numerals illustrating similar structures, components, materials and/or elements in different figures are indicated by similar reference numbers.
Figure 1 is a side view of the tool according to an embodiment of the present invention;
Figure 2 is a top view of the tool of Figure 1;
Figure 3 is an axonometric view of the tool of Figure 1;
Figure 4A is a detail of Figure 1 in which the beaks of the tool are in a clamped configuration;
Figure 4B shows the beaks of Figure 4A in an open configuration;
Figure 5 is a detail of Figure 2 in which the beaks of the tool are in a clamped configuration;
Figure 6 is a cross-sectional view of a cutting portion and an abutment portion of the beaks of the tool of Figure 1;
Figure 7 is a flow chart of a section procedure that can be performed by means of the tool according to the present invention, and
Figure 8 is a schematic top view of a cervical vertebra.

### DETAILED DESCRIPTION OF THE INVENTION

While the invention is susceptible to various modifications and alternative constructions, certain preferred embodiments are shown in the drawings and are described hereinbelow in detail. It is in any case to be noted that there is no intention to limit the invention to the specific embodiment illustrated, rather on the contrary, the invention intends covering all the modifications, alternative and equivalent constructions that fall within the scope of the invention as defined in the claims.

The use of "for example", "etc.", "or" indicates non-exclusive alternatives without limitation, unless otherwise indicated. The use of "includes" means "includes, but not limited to" unless otherwise stated.

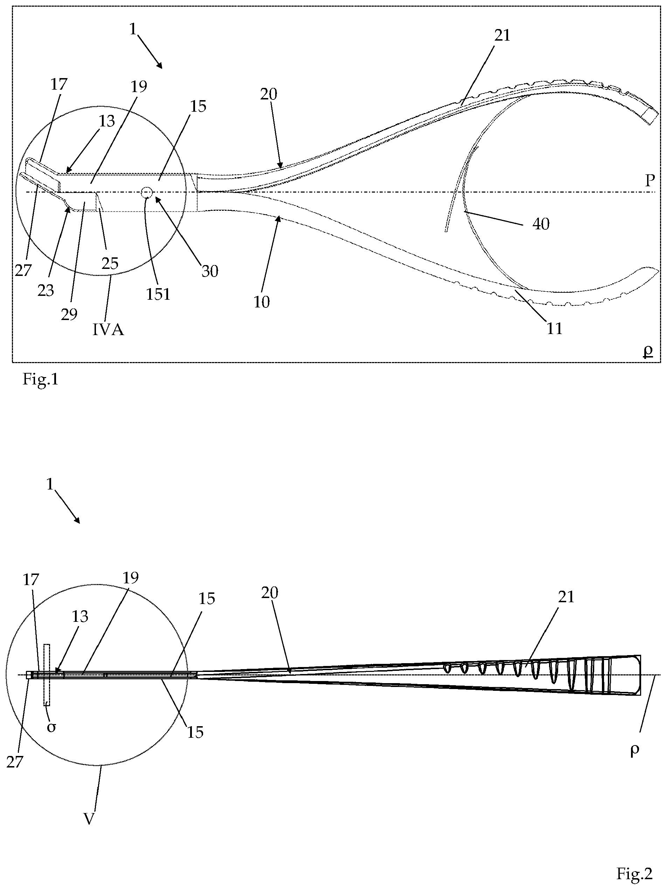

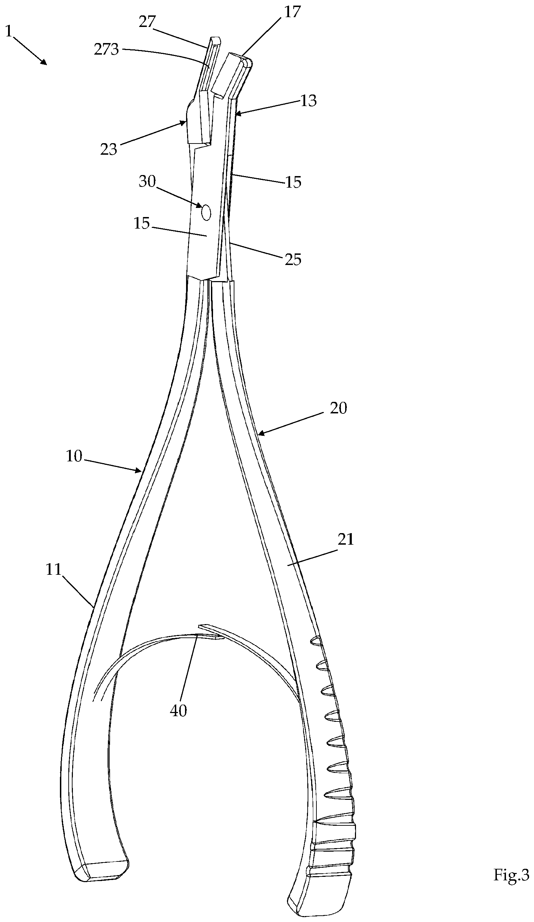

With reference to Figures 1 to 6, a tool for selectively sectioning the transverse processes of the cervical vertebrae is now described, simply referred to as tool 1 below, according to an embodiment of the present invention.

The tool comprises a first arm 10 and a second arm 20. Both the first arm 10 and the second arm 20 have a handle 11 and 21, respectively, and a beak 13 and 23, respectively. The handle 11 and 21 and the beak 13 and 23 of each arm 10 and 20 are substantially aligned along a main extension direction P of the tool 1 and are preferably formed as a single piece.

Furthermore, in the example shown in Figures 1 to 6, the first arm 10 comprises a pair of coupling portions 15, while the second arm 20 comprises a single coupling portion 25. The coupling portions 15 and 25 are interposed between the corresponding handle 11 and 21, and the corresponding beak 13 and 23. Each coupling portion 15 and 25 has a substantially plate-like structure and, in use, an orientation parallel to the rotation plane ρ. Each coupling portion 15 and 25 further comprises a through hole - of which only the hole 151 of the coupling portion is visible in Figures 1 to 6 - suitable for receiving a pin 30 in a rotatable manner - for example a pair of bolt screws, a rivet or other similar component. The two coupling portions 15 of the first arm are spaced apart to define a housing configured to receive the coupling portion 25 of the arm 20.

The first arm 10 and the second arm 20 are rotatably coupled one to the other by means of the pin 30. In the example considered, the coupling portion 25 of the second arm is inserted between the pair of coupling portions 15 of the first arm 10, so that the respective through holes are coaxial with each other. Then, the pin 30 is inserted into the through holes and locked in position.

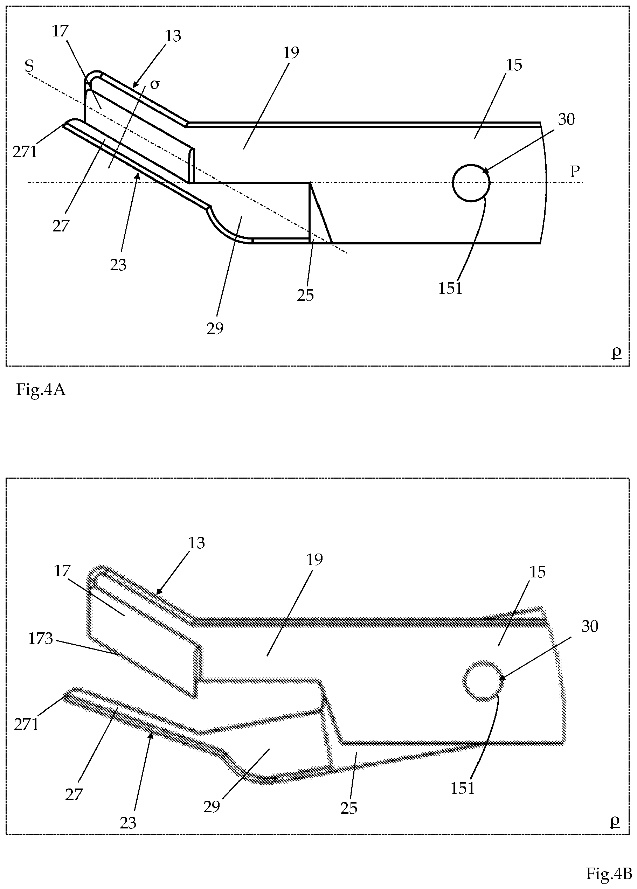

This allows a reciprocal rotation of the arms 10 and 20 in a rotation plane ρ. In particular, the arms 10 and 20 can rotate between a (first) clamped configuration and a (second) open configuration. In detail, in the clamped configuration, the beak 13 of the first arm 10 is in contact with the beak 23 of the second arm 20 (as can be seen in the detail of Figure 4A), while the handles 11 and 21 are proximal to each other. Differently, in the open configuration, the beak 13 of the first arm 10 is spaced from the beak 23 of the second arm 20 (as can be seen in the detail of Figure 4B), while the handles 11 and 21 are distal to each other.

In embodiments of the present invention, the beak 13 of the first arm 10 comprises a cutting portion 17. Conversely, the beak 23 of the second arm 20 comprises an abutment portion 27.

In the example considered, both the cutting portion 17 and the abutment portion 27 have a dimension of length that extends along a secondary extension direction S inclined at an angle included between 15° and 45°, preferably 30°, with respect to the main extension direction P of the tool 1 (as can be seen in Figure 4A).

Preferably, the beak 13 of the first arm 10 comprises a connecting portion 19 connected to the cutting portion 17 and to the coupling portion 15, therefore, closer to the handle 11 than the cutting portion 17. Similarly, the beak 23 of the second arm 20 comprises a connecting portion 29 connected to the abutment portion 27 and to the coupling portion 25, therefore, closer to the handle 21 than the abutment portion 17. As can be seen in Figures 1 to 3, said connecting portions 19 and 29 have a dimension of length extending along the main extension direction P.

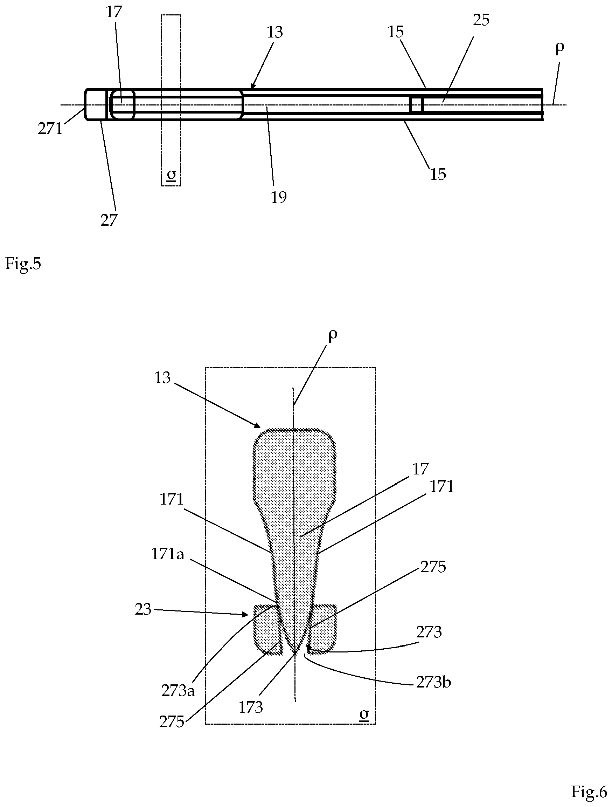

In particular, the cutting portion 17 has a structure wedge-like a elongated along the secondary extension direction S, with a substantially wedge-shaped section - in a section plane σ transversal to the rotation plane ρ and to the secondary extension direction S - delimited by two lateral surfaces 171 defining a cutting end 173 or edge (as can be seen in Figure 6). Preferably, both lateral surfaces 171 have a profile in the section plane σ having a convexity 171a - that is, a portion protruding outwards - in a position proximal to the cutting end 173 (as can be seen in Figure 6).

Conversely, the abutment portion 27 has a structure that is elongated along the secondary extension direction S substantially in the form of a parallelepiped. Preferably, the abutment portion 27 has a dimension of length greater than the cutting portion along the secondary extension direction S. Preferably, the abutment portion 27 comprises a free end 271 - along the secondary extension direction S - that is beveled or rounded.

Advantageously, the abutment portion 27 has a receptacle 273 configured to receive at least the cutting end 173 of the cutting portion 17 when the beak 13 of the first arm 10 is in contact with the beak 23 of the second arm 20 in the clamped configuration.

In the illustrated embodiment, the receptacle 273 is an elongated through slot comprising a first opening 273a exposed on the abutment portion 27 facing the beak 23 of the second arm 20 and a second opening 273b exposed on the abutment portion 27 opposite to the first opening 273a. Preferably, the receptacle 273 is configured to receive the cutting end 173 of the cutting portion 17 for its entire dimension of length.

Advantageously, when the beak 13 of the first arm 10 is in contact with the beak 23 of the second arm 20 in the clamped configuration, part of the cutting portion 17 is contained in the receptacle 273 and, preferably, the cutting end 173 of the cutting portion 17 does not protrude beyond the second opening 273b. For example, the cutting end 173 is flush with the second opening 273b when the arms 10 and 20 are in the clamped configuration (as can be seen in Figure 6).

In the embodiment considered, the lateral surfaces 171 of the cutting portion 17 are configured to come into contact with an internal side wall 275 of the abutment portion 27 delimiting the receptacle 273. Preferably, the contact between the internal side 275 and the lateral surfaces 171a takes place at the edge that delimits the first opening 273a of the through slot. Even more preferably, the contact between the side surfaces 171 of the cutting portion 17 and the side wall 275 of the abutment portion occurs in correspondence of the convexities 171a of the side surfaces 171.

In the embodiment considered in the figures, both the cutting portion 17 and the receptacle 273 of the abutment portion 27 - and, preferably, also the abutment portion 27 itself - have a substantially symmetrical structure with respect to the rotation plane ρ.

Optionally, the tool 1 may comprise an elastic metal arc system 40 fixed to the handles of the arms, in order to facilitate the return to the open configuration from the clamped configuration.

Experiments conducted by the Applicant have allowed the following optimal dimensioning ranges to be determined for the beaks 13 and 23 of the tool 1.

Both the cutting portion 17 of the beak 13 of the first arm 10 and the abutment portion 27 of the beak 23 of the second arm 20 are made with a maximum width dimension w - lying in the section plane σ and transversal to the rotation plane ρ - substantially between 5 mm and 1.5 mm (i.e. 1.5 mm ≤ *w* ≤ 5 mm), preferably substantially equal to 2 mm.

Moreover, the abutment portion 27 is made with a maximum depth dimension z - parallel to the rotation plane ρ - substantially between 1.5 mm and 1 mm (i.e. 1 mm ≤ z ≤ 1.5 mm), preferably substantially equal to 1.2 mm. Conversely, the cutting portion 17 has a maximum depth dimension z - lying in the section plane σ and parallel to the rotation plane ρ - substantially between 5 mm and 6.5 mm (i.e. 5 mm ≤ z ≤ 6.5 mm), preferably substantially equal to 6 mm.

Finally, the abutment portion 27 is made with a maximum length dimension *l* - lying in the rotation plane ρ and parallel to the secondary extension direction S - substantially comprised between 12 mm and 15 mm (i.e. 12 mm ≤ *l* ≤ 15 mm), preferably substantially equal to 13 mm. Conversely, the cutting portion 17 has a maximum length dimension *l* - lying in the rotation plane ρ and parallel to the secondary extension direction S - substantially comprised between 8 mm and 11 mm (i.e. 8 mm ≤ *l* ≤ 11 mm), preferably substantially equal to 10 mm.

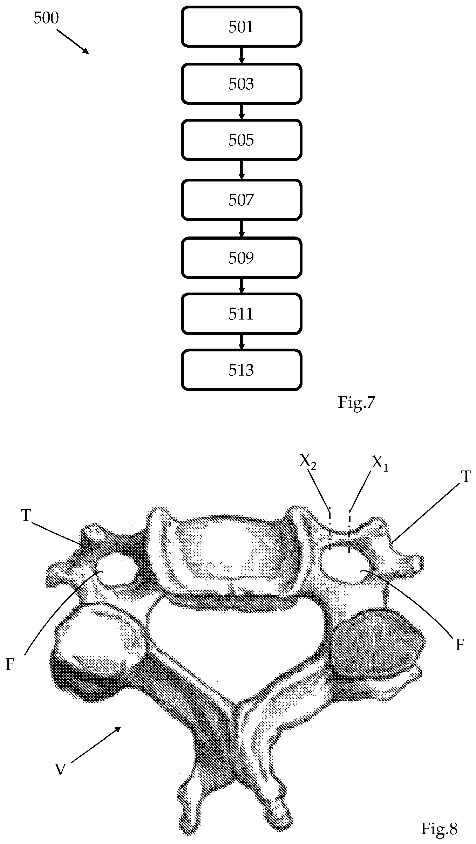

The tool 1 according to an embodiment of the present invention described above allows to perform a section procedure 500 (of which a flow chart is shown in Figure 7) of a transverse process T of a cervical vertebra V (shown schematically in Figure 8), for example, in the case of an autopsy, in a particularly simple way without compromising the structures - that is, the arterial and venous vertebral vessels - that run through the transverse foramen F.

Preferably, the tool is made of surgical grade steel - for example, austenitic stainless steel alloys, such as AISI 316, or martensitic stainless steel alloys, such as AISI 420 and AISI 440 -, titanium alloys or equivalent metals or metal alloys. In an embodiment forged with several materials, the cutting portion 17 and/or the abutment portion 27 are made of tool steel or sintered metal carbide. In addition or alternatively, the elastic metal arc system 40 is made of a harmonic steel.

Initially, one or both of the transverse processes T of one or more cervical vertebrae V are exposed by removing at least partially one or more tissues of the epidermis, of the cervical and muscular bands superimposed on said cervical vertebrae V (block 501).

Subsequently, the abutment portion 27 of the beak 23 of the second arm 20 of the tool 1 is inserted in the transverse foramen F near the internal wall of the transverse process T delimiting the transverse foramen F - for example, at the section line X₁ shown in Figure 8, while the tool is in the open configuration (block 503).

The abutment portion 27 is advanced until the transverse process T delimiting the transverse foramen F is completely superimposed on the receptacle 275 of the abutment portion (block 505). In other words, the abutment portion 27 is advanced until it passes through the entire transverse foramen F with at least the free end 271 protruding beyond said transverse foramen F.

The tool is then brought into the clamped configuration by acting on the handles 11 and 21 (block 507). The cutting portion 17 of the beak 13 of the first arm 10 then intercepts the transverse process T, causing first an incision and a sectioning of the same - along the section line Xi. Moreover, the wedge-shaped structure of the cutting portion 17 causes the away-movement of the bone margins generated by the incision during the clamping of the beaks 13 and 23. In this way the shearing action is precise and requires a very limited application force to reach the clamped configuration and, therefore, to obtain the complete sectioning of the transverse process T.

Moreover, the structures contained in the transverse foramen F are not damaged by the incision action, since the abutment portion 27 remains in position against the transverse process T while the cutting portion 17 is clamped.

Finally, the tool 1 is then moved away from the cervical vertebra V (block 509) having completed the operation at the section line Xi. The same procedure of introduction, cutting and away-movement described in the previous steps 503 - 509 is repeated for the same transverse process T of the same vertebra V in correspondence with a more medial plane - indicated by the section line X₂ in Figure 8 - in order to allow the isolation by section of a bone lamina (block 511). Finally, the bone lamina, isolated by virtue of the double section described above, is removed (block 513) and the structures contained in the transverse foramen F remain exposed through the incision produced by means of the tool 1, thereby allowing an accurate analysis thereof.

The invention thus conceived is susceptible to several modifications and variations, all falling within the scope of the inventive concept.

For example, in an alternative embodiment (not shown), the receptacle of the abutment portion has only the first opening turned towards the cutting portion, i.e. it is substantially a recess.

Again in a simplified embodiment (not shown), the cutting portion and the abutment portion have a length dimension substantially aligned with the main extension direction.

In a different embodiment (not shown), the cutting portion comprises a removable blade and a receptacle integral with the beak of the tool. In particular, the removable blade can be coupled to the receptacle so that at least one edge of the removable blade remains exposed and turned towards the abutment portion of the tool. For example, the receptacle comprises a shaped groove configured to receive a blade engaging portion, opposite to the blade edge and shaped to correspond to the shaped groove of the receptacle. Preferably, the engaging portion and the shaped groove are configured to stably couple through an engagement by interference fit and/ or comprise corresponding elements designed to snap engage with each other. In this way, it is possible to simply replace the blade of the tool in the event of wear or damage to the blade edge.

Moreover, there is nothing to prevent both arms from being made with a single coupling portion, which are draw near one another in order to make coaxial the respective through holes.

In addition or alternatively, the pin may be integral or formed as one piece with one of the two arms of the tool.

Moreover, all the details can be replaced by other technically equivalent elements.

For example, the handles of the tool may comprise rings for inserting fingers.

In practice, the materials used, as well as the contingent shapes and sizes, can be whatever according to the requirements without for this reason departing from the scope of protection of the following claims.

## Claims

1. Tool (1) for selectively sectioning the transverse processes of the cervical vertebrae comprising a first arm (10) and a second arm (20), each arm (10, 20) comprising a handle (11, 21) and a beak (13, 23) aligned along a main extension direction (P) of the tool (1), wherein the first arm (10) and the second arm (20) are rotatably coupled one to the other by means of a pin (30) in order to rotate in a rotation plane (ρ) between a first configuration in which the beak (13) of the first arm (10) is in contact with the beak (23) of the second arm (20) and a second configuration in which the beak (13) of the first arm (10) is spaced from the beak (23) of the second arm (20),
wherein the beak (13) of the first arm (10) comprises a cutting portion (17), said cutting portion (17) having a wedge-shaped section in a section plane (o) transversal to the rotation plane (ρ) of the arms (10, 20), and
the beak (23) of the second arm (20) comprises an abutment portion (27) having a receptacle (273) configured to receive a cutting end (173) of the cutting portion (17) when the beak (13) of the first arm (10) is in contact with the beak (23) of the second arm (20) in the first configuration,
**characterized in that**
the receptacle (273) of the abutment portion (27) comprises a through slot configured to receive the cutting end (173) of the cutting portion (17) for its entire dimension of length.

2. Tool (1) according to the claims 1, wherein both the cutting portion (17) and the receptacle (273) of the abutment portion (27) have a symmetrical structure with respect to the rotation plane (ρ) of the arms (10, 20) of the tool (1).

3. Tool (1) according to any one of the preceding claims, wherein the cutting portion (17) comprises a pair of side surfaces (171) which joins together to define the cutting end (173), and when the beak (13) of the first arm (10) is in contact with the beak (23) of the second arm (20) in the second configuration, said lateral surfaces (171) are configured to come into contact with an internal side wall (275) of the abutment portion (27), said internal side wall (275) delimiting the receptacle (273).

4. Tool (1) according to claim 3, wherein a profile of each of said side surfaces (171) of the cutting portion (27) has a convexity (171a) in a position proximal to the cutting end (173), the contact between the side surfaces (171) of the cutting portion (17) and the side wall (275) of the abutment portion (27) occurring in correspondence of said convexity (171a).

5. Tool (1) according to claim 3 or 4, wherein the receptacle (273) comprises a through slot comprising a first opening (273a) exposed on the abutment portion (27) facing the beak (13) of the first arm (10) and a second opening (273b) exposed on the abutment portion (27) opposite to the first opening (273a), and when the beak (13) of the first arm (10) is in contact with the beak (23) of the second arm (20) in the second position, the cutting end (123) of the cutting portion (17) does not protrude beyond the second opening (273b) of the through slot.

6. Tool (1) according to any one of the preceding claims, wherein the cutting portion (17) and the abutment portion (27) have a dimension of length that extends along a secondary extension direction (S) inclined at an angle included between 15° and 45°, preferably equal to 30°, with respect to the main extension direction (P) of the tool (1).

7. Tool (1) according to claim 6, wherein the beak (13) of the first arm (10) comprises a connecting portion (19) connected to the cutting portion (17) and proximal to the handle (11) of the first arm (10), and wherein the beak (23) of the second arm (20) comprises a connecting portion (29) connected to the abutment portion (27) and proximal to the handle (21) of the second arm (20), said connecting portions (19, 29) having a dimension of length extending along the main extension direction (P).

8. The tool (1) according to any one of the preceding claims, wherein the first arm (10) comprises a pair of coupling portions (15) interposed between the beak (13) and the handle (11), and wherein the second arm (20) comprises a single coupling portion (25) interposed between the beak (23) and the handle (21), wherein the two coupling portions (15) of the first arm (10) are spaced apart so as to define a housing configured to receive the coupling portion (25) of the second arm (20), and in which each coupling portion (15, 25) comprises a through hole configured to be coaxial with the through holes of the other coupling portions (15, 25) and for receiving said pin (30) in a rotatable manner.

9. Tool (1) according to any one of the preceding claims, wherein the abutment portion (27) comprises a beveled or rounded free end (271).

10. Tool (1) according to any one of the preceding claims, wherein both the cutting portion (17) and the abutment portion (27) have a maximum width dimension, lying in the section plane (o), between 1.5 mm and 5 mm, preferably equal to 2 mm, and wherein the abutment portion (27) is made with a maximum length dimension, lying in the rotation plane ρ, substantially comprised between 12 mm and 15 mm, preferably substantially equal to 13 mm, and wherein the cutting portion (17) has a maximum length dimension, lying in the rotation plane ρ, substantially comprised between 8 mm and 11 mm, preferably substantially equal to 10 mm.

11. Tool (1) according to any one of the preceding claims, wherein the abutment portion (27) has a maximum depth dimension parallel to the rotation plane (ρ) between 1.5 mm and 1 mm, preferably equal to 1.2 mm, and wherein the cutting portion (17) has a dimension of maximum depth parallel to the rotation plane (ρ) comprised between 5 mm and 6.5 mm, preferably equal to 6 mm.

12. Tool (1) according to any one of the preceding claims, wherein the cutting portion (17) comprises a removable blade and a receptacle integral with the beak (13) of the first arm (10), wherein the removable blade is coupled in a removable manner with the receptacle.

## Patentansprüche

1. Werkzeug (1) zum selektiven Schneiden der Querfortsätze der Halswirbelsäule, umfassend einen ersten Arm (10) und einen zweiten Arm (20), jeder Arm (10, 20) umfassend einen Griff (11, 21) und einen Schnabel (13, 23) ausgerichtet entlang einer Haupterstreckungsrichtung (P) des Werkzeugs (1), wobei der erste Arm (10) und der zweite Arm (20) durch einen Stift (30) drehbar miteinander gekoppelt sind, um sich in einer Rotationsebene (ρ) zwischen einer ersten Konfiguration, in welcher der Schnabel (13) des ersten Arms (10) in Kontakt mit dem Schnabel (23) des zweiten Arms (20) ist, und einer zweiten Konfiguration, in welcher der Schnabel (13) des ersten Arms (10) von dem Schnabel (23) des zweiten Arms (20) beabstandet ist, zu drehen,
wobei der Schnabel (13) des ersten Arms (10) ein Schneideabschnitt (17) umfasst, wobei besagter Schneideabschnitt (17) einen keilförmigen Bereich in einer Schnittebene (σ) quer zu der Rotationsebene (ρ) der Arme (10, 20) hat, und
wobei der Schnabel (23) des zweiten Arms (20) einen Anschlagabschnitt (27) mit einer Aufnahme (273) aufweist, die dazu eingerichtet ist, ein Schneidende (173) des Schneideabschnitts (17) aufzunehmen, wenn der Schnabel (13) des ersten Arms (10) in der ersten Konfiguration in Kontakt mit dem Schnabel (23) des zweiten Arms (20) ist,
**dadurch gekennzeichnet, dass**
die Aufnahme (273) des Anschlagabschnitts (27) einen Durchgangsschlitz umfasst, der dazu eingerichtet ist, das Schneidende (173) des Schneideabschnitts (17) über seine gesamte Längendimension aufzunehmen.

2. Werkzeug (1) nach Anspruch 1, **dadurch gekennzeichnet, dass** sowohl der Schneideabschnitt (17) als auch die Aufnahme (273) des Anschlagabschnitts (27) eine symmetrische Struktur in Bezug auf die Rotationsebene (ρ) der Arme (10, 20) des Werkzeugs (1) aufweisen.

3. Werkzeug (1) nach einem der vorhergehenden Ansprüche, wobei der Schneideabschnitt (17) ein Paar von Seitenflächen (171) umfasst, welches sich zusammenfügt, um das Schneidende (173) zu definieren, und wenn der Schnabel (13) des ersten Arms (10) in der zweiten Konfiguration in Kontakt mit dem Schnabel (23) des zweiten Arms (20) ist, besagte Seitenflächen (171) dazu eingerichtet sind, mit einer inneren Seitenwand (275) des Anschlagabschnitts (27) in Kontakt kommen, wobei besagte Seitenwand (275) die Aufnahme (273) begrenzt.

4. Werkzeug (1) nach Anspruch 3, wobei ein Profil von jeder der besagten Seitenflächen (171) des Schneideabschnitts (27) eine Konvexität (171a) in einer Position nahe dem Schneidende (173) hat, wobei der Kontakt zwischen den Seitenflächen (171) des Schneideabschnitts (17) und der Seitenwand (275) des Anschlagabschnitts (27) in Übereinstimmung mit besagter Konvexität (171a) auftritt.

5. Werkzeug (1) nach Anspruch 3 oder 4, wobei die Aufnahme (273) einen Durchgangsschlitz umfassend eine erste Öffnung (273a), die an dem dem Schnabel (13) des ersten Arms (10) zugewandten Anschlagabschnitt (27) freiliegt, und eine zweite Öffnung (273b), die an dem Anschlagabschnitt (27) gegenüber der ersten Öffnung (273a) freiliegt, umfasst und wenn der Schnabel (13) des ersten Arms (10) in der zweiten Position in Kontakt mit dem Schnabel (23) des zweiten Arms (20)ist, das Schneidende (123) des Schneideabschnitts (17) nicht über die zweite Öffnung (273b) des Durchgangsschlitzes hinausragt.

6. Werkzeug (1) nach einem der vorhergehenden Ansprüche, wobei der Schneideabschnitt (17) und der Anschlagabschnitt (27) eine Längendimension haben, welche sich entlang einer sekundären Erstreckungsrichtung (S) erstreckt, die in einem Winkel zwischen 15° und 45°, vorzugsweise gleich 30°, in Bezug auf die Haupterstreckungsrichtung (P) des Werkzeugs (1) geneigt ist.

7. Werkzeug (1) nach Anspruch 6, wobei der Schnabel (13) des ersten Arms (10) einen Verbindungsabschnitt (19) umfasst, der mit dem Schneideabschnitt (17) verbunden ist und nahe des Griffs (11) des ersten Arms (10) ist, und wobei der Schnabel (23) des zweiten Arms (20) einen Verbindungsabschnitt (29) umfasst, der mit dem Anschlagabschnitt (27) verbunden ist und nahe des Griffs (21) des zweiten Arms (20) ist, wobei besagte Verbindungsabschnitte (19, 29) eine sich entlang der Haupterstreckungsrichtung (P) erstreckende Längendimension haben.

8. Werkzeug (1) nach einem der vorhergehenden Ansprüche, wobei der erste Arm (10) ein Paar von zwischen dem Schnabel (13) und dem Griff (11) angeordnete Kupplungsabschnitte (15) umfasst, und wobei der zweite Arm (20) einen einzigen zwischen dem Schnabel (23) und dem Griff (21) angeordneten Kupplungsabschnitt (25) umfasst, wobei die zwei Kupplungsabschnitte (15) des ersten Arms (10) so beabstandet sind, dass ein Gehäuse definiert wird, das dazu eingerichtet ist, den Kupplungsabschnitt (25) des zweiten Arms (20) aufzunehmen, und in dem jeder Kupplungsabschnitt (15, 25) ein Durchgangsloch aufweist, das dazu eingerichtet ist, koaxial mit den Durchgangslöchern der anderen Kupplungsabschnitte (15, 25) zu sein und den Stift (30) drehbar aufzunehmen.

9. Werkzeug (1) nach einem der vorhergehenden Ansprüche, wobei der Anschlagabschnitt (27) ein abgeschrägtes oder abgerundetes freies Ende (271) aufweist.

10. Werkzeug (1) nach einem der vorhergehenden Ansprüche, wobei sowohl der Schneideabschnitt (17) als auch der Anschlagabschnitt (27) eine maximale Breitendimension, die in der Schnittebene (σ) liegt, zwischen 1. 5 mm und 5 mm, vorzugsweise gleich 2 mm, aufweisen, und wobei der Anschlagabschnitt (27) mit einer maximalen Längendimension, die in der Rotationsebene ρ liegt, gemacht ist, die im Wesentlichen zwischen 12 mm und 15 mm liegt, vorzugsweise im Wesentlichen gleich 13 mm ist, und wobei der Schneideabschnitt (17) eine maximale Längendimension, die in der Rotationsebene ρ liegt, hat, die im Wesentlichen zwischen 8 mm und 11 mm liegt, vorzugsweise im Wesentlichen gleich 10 mm ist.

11. Werkzeug (1) nach einem der vorhergehenden Ansprüche, **dadurch gekennzeichnet, dass** der Anschlagabschnitt (27) eine maximale Tiefendimension parallel zur Rotationsebene (ρ) zwischen 1,5 mm und 1 mm, vorzugsweise gleich 1,2 mm, hat, und dass der Schneideabschnitt (17) eine maximale Tiefendimension parallel zur Rotationsebene (ρ) zwischen 5 mm und 6,5 mm, vorzugsweise gleich 6 mm, hat.

12. Werkzeug (1) nach einem der vorhergehenden Ansprüche, wobei der Schneideabschnittl (17) eine abnehmbare Klinge und eine mit dem Schnabel (13) des ersten Arms (10) einstückige Aufnahme umfasst, wobei die abnehmbare Klinge abnehmbar mit der Aufnahme verbunden ist.s

## Revendications

1. - Outil (1) pour sectionner de manière sélective les apophyses transverses des vertèbres cervicales, comprenant un premier bras (10) et un second bras (20), chaque bras (10, 20) comprenant une poignée (11, 21) et un bec (13, 23) alignés le long d'une direction d'extension principale (P) de l'outil (1), le premier bras (10) et le second bras (20) étant accouplés de manière rotative l'un à l'autre au moyen d'un axe (30) afin de tourner dans un plan de rotation (ρ) entre une première configuration dans laquelle le bec (13) du premier bras (10) est en contact avec le bec (23) du second bras (20) et une seconde configuration dans laquelle le bec (13) du premier bras (10) est espacé du bec (23) du second bras (20),
le bec (13) du premier bras (10) comprenant une partie de coupe (17), ladite partie de coupe (17) ayant une section en forme de coin dans un plan de coupe (σ) transversal au plan de rotation (ρ) des bras (10, 20), et
le bec (23) du second bras (20) comprenant une partie de butée (27) ayant un réceptacle (273) configuré pour recevoir une extrémité de coupe (173) de la partie de coupe (17) lorsque le bec (13) du premier bras (10) est en contact avec le bec (23) du second bras (20) dans la première configuration,
**caractérisé par le fait que** le réceptacle (273) de la partie de butée (27) comprend une fente traversante configurée pour recevoir l'extrémité de coupe (173) de la partie de coupe (17) sur toute sa dimension de longueur.

2. - Outil (1) selon les revendications 1, dans lequel la partie de coupe (17) et le réceptacle (273) de la partie de butée (27) ont une structure symétrique par rapport au plan de rotation (ρ) des bras (10, 20) de l'outil (1).

3. - Outil (1) selon l'une quelconque des revendications précédentes, dans lequel la partie de coupe (17) comprend une paire de surfaces latérales (171) qui se rejoignent pour définir l'extrémité de coupe (173) et, lorsque le bec (13) du premier bras (10) est en contact avec le bec (23) du second bras (20) dans la seconde configuration, lesdites surfaces latérales (171) sont configurées pour entrer en contact avec une paroi latérale interne (275) de la partie de butée (27), ladite paroi latérale interne (275) délimitant le réceptacle (273).

4. - Outil (1) selon la revendication 3, dans lequel un profil de chacune desdites surfaces latérales (171) de la partie de coupe (27) a une convexité (171a) dans une position proximale à l'extrémité de coupe (173), le contact entre les surfaces latérales (171) de la partie de coupe (17) et la paroi latérale (275) de la partie de butée (27) se produisant en correspondance de ladite convexité (171a).

5. - Outil (1) selon la revendication 3 ou 4, dans lequel le réceptacle (273) comprend une fente traversante comprenant une première ouverture (273a) exposée sur la partie de butée (27) faisant face au bec (13) du premier bras (10) et une seconde ouverture (273b) exposée sur la partie de butée (27) opposée à la première ouverture (273a) et, lorsque le bec (13) du premier bras (10) est en contact avec le bec (23) du second bras (20) dans la seconde position, l'extrémité de coupe (123) de la partie de coupe (17) ne fait pas saillie au-delà de la seconde ouverture (273b) de la fente traversante.

6. - Outil (1) selon l'une quelconque des revendications précédentes, dans lequel la partie de coupe (17) et la partie de butée (27) ont une dimension de longueur qui s'étend le long d'une direction d'extension secondaire (S) inclinée à un angle compris entre 15° et 45°, de préférence égal à 30°, par rapport à la direction d'extension principale (P) de l'outil (1).

7. - Outil (1) selon la revendication 6, dans lequel le bec (13) du premier bras (10) comprend une partie de liaison (19) reliée à la partie de coupe (17) et proximale à la poignée (11) du premier bras (10), et dans lequel le bec (23) du second bras (20) comprend une partie de liaison (29) reliée à la partie de butée (27) et proximale à la poignée (21) du second bras (20), lesdites parties de liaison (19, 29) ayant une dimension de longueur s'étendant le long de la direction d'extension principale (P).

8. - Outil (1) selon l'une quelconque des revendications précédentes, dans lequel le premier bras (10) comprend une paire de parties d'accouplement (15) interposées entre le bec (13) et la poignée (11), et dans lequel le second bras (20) comprend une seule partie d'accouplement (25) interposée entre le bec (23) et la poignée (21), les deux parties d'accouplement (15) du premier bras (10) étant espacées de façon à définir un logement configuré pour recevoir la partie d'accouplement (25) du second bras (20), et chaque partie d'accouplement (15, 25) comprend un trou traversant configuré pour être coaxial aux trous traversants des autres parties d'accouplement (15, 25) et pour recevoir ledit axe (30) d'une manière rotative.

9. - Outil (1) selon l'une quelconque des revendications précédentes, dans lequel la partie de butée (27) comprend une extrémité libre biseautée ou arrondie (271).

10. - Outil (1) selon l'une quelconque des revendications précédentes, dans lequel à la fois la partie de coupe (17) et la partie de butée (27) ont une dimension de largeur maximale, s'étendant dans le plan de coupe (σ), comprise entre 1,5 mm et 5 mm, de préférence égale à 2 mm, et dans lequel la partie de butée (27) est réalisée avec une dimension de longueur maximale, dans le plan de rotation (ρ), sensiblement comprise entre 12 mm et 15 mm, de préférence sensiblement égale à 13 mm, et dans lequel la partie de coupe (17) a une dimension de longueur maximale, s'étendant dans le plan de rotation (ρ), sensiblement comprise entre 8 mm et 11 mm, de préférence sensiblement égale à 10 mm.

11. - Outil (1) selon l'une quelconque des revendications précédentes, dans lequel la partie de butée (27) a une dimension de profondeur maximale parallèle au plan de rotation (ρ) comprise entre 1,5 mm et 1 mm, de préférence égale à 1,2 mm, et dans lequel la partie de coupe (17) a une dimension de profondeur maximale parallèle au plan de rotation (ρ) comprise entre 5 mm et 6,5 mm, de préférence égale à 6 mm.

12. - Outil (1) selon l'une quelconque des revendications précédentes, dans lequel la partie de coupe (17) comprend une lame amovible et un réceptacle solidaire du bec (13) du premier bras (10), la lame amovible étant accouplée d'une manière amovible au réceptacle.
